# EUROPEAN PATENT SPECIFICATION

(11) **EP 4 208 094 B1**
(45) Date of publication and mention of the grant of the patent: **05.11.2025**
(21) Application number: 21791497.7
(22) Date of filing: 02.09.2021
(51) Int. Cl.: A61B 5/20, A61B 5/00

(54) **FOLEY CATHETER COMPRISING OXYGEN SENSOR AND PUMP**
FOLEY-KATHETER MIT SAUERSTOFFSENSOR UND PUMPE
CATHÉTER DE FOLEY COMPRENANT UN CAPTEUR D'OXYGÈNE ET UNE POMPE

(30) Priority: 04.09.2020 US 202063074763 P
(43) Date of publication of application: 12.07.2023
(73) Proprietor: Covidien LP, Mansfield, MA 02048 (US)
(72) Inventor: DOVE, Jacob D., Boulder, Colorado 80301 (US); AASMUL, Soren, 2300 Copenhagen (DK); SMITH, William S., Boulder, Colorado 80301 (US); MILLER, David J., Boulder, Colorado 80301 (US); KRISTENSEN, Jesper Svenning, 2300 Copenhagen (DK)
(74) Representative: Marks & Clerk LLP
(86) International application number: PCT/US2021/048933
(87) International publication number: WO 2022/051550

(56) References cited:
- US-A1- 2017 136 209
- US-A1- 2019 069 831
- US-A1- 2020 054 800

## Description

This application claims the benefit of U.S. Provisional Application No. 63/074,763, entitled, "ACUTE KIDNEY INJURY MONITORING" and filed September 4, 2020.

### TECHNICAL FIELD

This disclosure relates to patient monitoring.

### BACKGROUND

Medical devices, such as catheters, may be used to assist a patient in voiding their bladder. In some instances, such catheters may be used during and/or after surgery. In the case of using a catheter to assist a patient in voiding their bladder, a Foley catheter is a type of catheter that may be used for longer time periods than a non-Foley catheter. Some Foley catheters are constructed of silicon rubber and include an anchoring member, which may be an inflatable balloon, that may be inflated in a patient's bladder so a proximal end of the catheter does not slip out of the patient's bladder.

Foley catheters are known from e.g. US 2017/136209 and US 2019/069831.

### SUMMARY

In general, the disclosure devices, systems, and techniques for renal monitoring of a patient, e.g., to monitor the oxygenation of the kidneys and detect one or conditions indicative of acute kidney injury of the patient. In examples described herein, a dissolved oxygen sensor is configured to generate a signal indicative of an amount of oxygen dissolved in urine of a patient (e.g., urinary oxygen tension (uPO₂ or PuO₂)), from which a clinician or a device may be able to determine oxygenation status of the one or both kidneys of the patient. In some cases, oxygen diffusion from an environment external to the bladder or from the bladder wall may cause an amount of oxygen in the urine to change before it can be sensed by the dissolved oxygen sensor, such that the urine sensed by the dissolved oxygen sensor may not be indicative of the oxygenation status of one or both kidneys. For example, if a Foley catheter is used to remove urine from the bladder, oxygen diffusion through a wall of the Foley catheter may occur at the relatively low flow rates that are typical for urine output for catheterized patients. In examples described herein, to minimize the impacts of the oxygen diffusion into the urine from an environment external to the bladder or from the bladder wall, a sensing system includes a pump configured to move the urine from the bladder to facilitate active sampling of the urine and enable a device to more accurately determine an amount of oxygen dissolved in the urine.

In one example, this disclosure is directed to a method including: pumping, by a pump, a fluid to direct the fluid to a dissolved oxygen sensor; and outputting, by the dissolved oxygen sensor, a signal indicative of an amount of dissolved oxygen in the fluid.

In another example, this disclosure is directed to a system including: a pump configured to pump fluid to direct the fluid a dissolved oxygen sensor; and the dissolved oxygen sensor configured to output a signal indicative of an amount of dissolved oxygen in the fluid.

In another example, this disclosure is directed to a triple lumen Foley catheter including: a pump configured to pump urine from a bladder of a patient to direct the urine to a dissolved oxygen sensor; and the dissolved oxygen sensor configured to output a signal indicative of an amount of dissolved oxygen in the urine.

A system in accordance with the invention is defined in claim 1.

The details of one or more examples are set forth in the accompanying drawings and the description below. Other features, objects, and advantages will be apparent from the description and drawings, and from the claims.

### BRIEF DESCRIPTION OF DRAWINGS

FIG. 1 is a conceptual diagram illustrating an example catheter.
FIG. 2 is a conceptual diagram illustrating an example cross-sectional view of the catheter of FIG. 1, the cross-sections being taken along lines 2-2 of FIG. 1.
FIG. 3 is a block diagram of an example external device that may be used with a medical device.
FIG. 4 is a graphic diagram illustrating pO₂ measurements of water that was initially set to ~43 mmHg and allowed to flow through a silicone Foley catheter at different flow rates.
FIG. 5 is a conceptual diagram of an example pO₂ sensor coupled to a Foley catheter.
FIG. 6 is a conceptual diagram of another example pO₂ sensor within a lumen of a Foley catheter.
FIGS. 7A and 7B are conceptual diagrams of an example active valve located in or about a drainage lumen of a catheter.
FIG. 8 is a flow diagram illustrating an example active sampling technique.

### DETAILED DESCRIPTION

Acute kidney injury (AKI) is a complication that may occur after some medical procedures, such as some cardiac surgeries, e.g., coronary artery bypass grafting (CABG). AKI may also occur after other surgeries that are lengthy and involve significant blood loss or fluid shifts. For example, a body of a surgery patient may alter where their blood is directed which may lead to hypoxia of a kidney, which may cause an ischemia reperfusion injury in a kidney of the patient. This ischemia reperfusion injury may cause degradation of renal function of the patient. The degradation of renal function may cause an accumulation of waste products in the bloodstream, which may delay the patient's recovery from the surgery and lead to more extended hospital stays, require dialysis, and may even lead to further complications, such as chronic kidney disease.

The present disclosure describes example techniques, devices, and systems that facilitate monitoring of kidney function of patients based on an oxygen content of a fluid (e.g., urine) removed from a bladder of the patient. As discussed in further detail below, in some examples, a medical device includes a dissolved gas sensor, such as a dissolved oxygen sensor configured to sense an amount of oxygen dissolved in the urine (e.g., urinary oxygen tension (uPO₂ or PuO₂)) in the bladder and/or sense urine output of a patient (e.g., rate of urine production), from which a clinician or a device may be able to determine oxygenation status of the one or both kidneys of the patient.

The monitoring of kidney function may help reduce occurrences of AKI by providing clinicians with a real-time and continuous assessment of the risk that a specific patient may develop AKI. This may facilitate a clinician intervening prior to the patient developing AKI. For example, a clinician may initiate or make changes to hemodynamic management (e.g., blood pressure management, fluid management, blood transfusions, and the like), make changes to cardiopulmonary bypass machine settings, or avoid providing nephrotoxic drugs. Post operatively, a clinician may intervene with a Kidney Disease: Improving Global Outcomes (KDIGO) bundle or an AKI care bundle, which may be predetermined set of guidelines and practices for the clinician to follow.

While systemic vital signs like cardiac output, blood pressure, and hematocrit may be useful for monitoring the kidney function of a patient (also referred to herein as renal monitoring), it may also be useful to monitor the oxygenation status of the kidneys in order to limit, reduce the severity of, or even prevent the risk of AKI. Renal monitoring can also be used to inform and guide hemodynamic management and fluid balance. The amount of dissolved oxygen in a urine of a patient may be indicative of kidney function, kidney perfusion, or kidney health. Dissolved oxygen in a patient's urine and bladder may correlate to perfusion and/or oxygenation of the kidneys, which is indicative of kidney function. Accurate monitoring of the oxygenation status of the kidneys can be challenging due to the inaccessibility of the kidneys. Near-Infrared spectroscopy (NIRS) measures regional oxygen saturation, and has some utility in babies and relatively slender adults in measuring oxygenation of the kidneys, but may not have the depth of penetration and specificity required for many adults. As described in further detail below, the example devices, systems, and techniques described herein help facilitate monitoring of the amount of dissolved oxygen in a urine by at least pumping urine in a direction away from the bladder and towards the oxygen sensor to facilitate active sampling of the urine.

While urine, bladders, and AKI are primarily referred to herein to describe the example medical devices, in other unclaimed examples, the medical devices may be used with other target locations in a patient, such as intravascular locations, and to monitor fluids of interest other than urine and/or other patient conditions other than kidney function. In addition, while catheters are primarily referred to herein, in other unclaimed examples, the medical device can have another configuration.

A medical device includes at least one sensor configured to sense a parameter of urine in the case of kidney function monitoring. In some unclaimed examples, the at least one sensor configured to sense a parameter of a fluid of interest may not be a part of the medical device, but be distal to a distal end of the medical device.

Parameters of interest sensed by a sensor described herein include, an amount of dissolved oxygen, and further optionally urine flow rate, urine concentration, urine electrical conductivity, urine specific gravity, urine biomarkers, amount of dissolved carbon dioxide in the urine, urine pH, bladder or abdominal pressure, bladder temperature, urine color, urine turbidity, urine creatinine, urine electrical conductivity, urine sodium, or motion from an accelerometer or other motion sensor. In some cases, it may be desirable to sense one or more of these parameters relatively close to the kidneys as possible because when sensors are positioned further away from the kidneys, the risk of introducing noise or losing signal strength increases and/or the risk of the concentration or integrity of a substance of interest in the fluid of interest (e.g., urine) changing prior to being sensed by the sensor may increase. For example, an electrical, optical or radio frequency signal representative of a parameter sensed close to the kidneys, may be affected by noise and/or loss of signal strength as the signal travels from a sensor close to the kidneys to a device that may process the signal and display information regarding the sensed parameter. As another example, in the case of a Foley catheter, it may be desirable to sense one or more of these parameters at the proximal end of the Foley catheter (e.g., in the bladder of the patient). However, placing these sensors at the proximal end of the catheter may increase the size and stiffness of the catheter and, as a result, may undermine the patient comfort or deliverability of the catheter. By design, a Foley catheter is configured to be small and flexible, such that it can be inserted through the urethra and into the bladder of a patient. If a Foley catheter were stiffer, then it may be more difficult to comfortably insert the catheter into the bladder of the patient. In some examples, an external device may estimate a parameter inside a bladder of a patient based on sensing distal to the patient.

As used herein, "sense" may include detect and/or measure." As used herein, "proximal" is used as defined in Section 3.1.4 of ASTM F623-19, Standard Performance Specification for Foley Catheter. That is, the proximal end of a catheter is the end closest to the patient when the catheter is being used by the patient. The distal end is therefore the end furthest from the patient. In some examples, "prevent" may mean completely prevent or partially prevent (e.g., effectively prevent), such as by restricting, inhibiting, impeding, or hindering. For example, to prevent the flow of a substance through a lumen may mean that the substance may enter an end of the lumen but all of the substance may not exit the other end of the lumen.

A medical device includes an oxygen sensor configured to sense oxygen content in a fluid sample and generate a signal indicative of the oxygen content. The oxygen content indicates an amount of oxygen dissolved in the fluid (e.g., oxygen partial pressure (pO₂), uPO₂, or PuO₂). The signal from the oxygen sensor may enable a clinician or a device to determine the oxygenation status of one or both kidneys of the patient.

The amount of dissolved oxygen in urine of a patient may be indicative of kidney function or kidney health of the patient. For example, dissolved oxygen in urine of the patient in the bladder may correlate to perfusion and/or oxygenation of the kidneys, which is indicative of kidney performance or kidney function. However, dissolved oxygen can be relatively difficult to measure. One way to measure dissolved oxygen is by fluorescence or luminescence lifetime sensor(s). The rate of decay of glow is indicative of the level of oxygen in a patient's urine. To more accurately measure the level of oxygen in urine of a patient, it may be desirable to take the measurement prior to any significant modification in the oxygen content in the urine, e.g., as close to the kidneys as possible. However, it may not be feasible to place a dissolved oxygen sensor at the proximal end of the catheter as doing so may increase cost, size, and flexibility of the catheter.

Some Foley catheters include an elongated body made from a silicone rubber that is relatively permeable to oxygen. Thus, as a fluid flows through a drainage lumen of the Foley catheter from a proximal opening to the drainage lumen to a distal opening to the drainage lumen, some oxygen may permeate from the surrounding environment through the walls of the elongated body into urine in the drainage lumen or dissipate through the walls of the elongated body and into a surrounding environment. In some examples, to minimize the exchange of oxygen or other substance of interest between the drainage lumen and the exterior of the catheter, it may be desirable to minimize transit time of urine (or other fluid) from the bladder of a patient to a sensor on the distal portion of such a Foley catheter or distal to a distal end of the Foley catheter. Slower fluid transit times through the catheter lumen may result in erroneous or skewed measurements as the oxygen may permeate through the walls of the Foley catheter into the urine or dissipate from the urine through the walls of the Foley catheter as the urine travels from the bladder through the lumen. For example, oxygen may permeate into the urine through the walls of the Foley catheter as the urine travels through the lumen from the bladder to the sensor. The oxygen may, for example, dissipate into, out of, or permeate from other tissues in or near the urinary tract and the atmosphere outside of the urinary tract. In some examples, the systems and catheters described herein enable a sensor to sense a substance of interest in a fluid despite being positioned relatively far away from the fluid source, such as a bladder. Thus, it may be desirable to have a Foley catheter that includes material relatively impermeable to oxygen and/or to decrease the transit time of urine traveling through the bladder to the sensor.

In accordance with examples of this disclosure, rather than integrating all of the desired sensors in the proximal portion of an elongated body of a catheter (e.g., the portion that is to be inserted into the bladder of the patient or otherwise introduced in a patient), one or more sensors may be positioned anywhere along the elongated body (e.g., on the proximal portion or a distal portion) or distal to a distal end of the elongated body. The distal portion of the elongated body may include, for example, the portion intended to remain outside of the patient when the proximal portion is introduced in the patient. By locating sensors at the distal portion of the catheter or distal to a distal end of the elongated body, the sensors may be larger, may rely upon relatively more electrical and/or optical connections and the catheter itself may be smaller and more flexible than it would have been had all the sensors been positioned at the proximal portion of the catheter.

A pump (e.g., a device configured to move fluid) is configured to actively move fluid towards an oxygen sensor (or other sensor) from a bladder of a patient (or from another source) to enable active sampling of the fluid. The pump can be, for example, located in a lumen of a catheter. By actively sampling, this disclosure refers to sensing a parameter of the fluid while the fluid is being pumped by a pump or other device. In contrast to passive sampling, e.g., relying on gravity, pressure, or other naturally occurring forces to move the fluid towards the sensor, active sampling may decrease the transit time of the fluid from the fluid source to the sensor, which can result in a more accurate sensing of the dissolved oxygen content or other parameter of interest in the fluid. By pumping the fluid so as to cause the fluid to flow at a higher flow rate, transit time of the fluid from the bladder to the sensor sampling the fluid may be reduced, which may facilitate more accurate sensing of one or more parameters of interest.

In some examples, the pump is configured to pump fluid through a lumen (e.g., a drainage lumen or a separate lumen) from a bladder of a patient to an oxygen sensor. In other examples, the pump can be configured to pump fluid from a drainage lumen of a catheter to a smaller diameter sampling lumen. Other pumping arrangements can also be used.

In some examples, when not actively sampling the fluid, the pump is configured to reduce or prevent flow through the lumen of the catheter in a direction away from the fluid source. In addition, or instead, a catheter can include a check valve located in or about a lumen of the elongated body and configured to prevent a back flow of a fluid, such as urine. The check valve can be, for example, a one-way valve configured to permit fluid flow in a distal direction through the lumen and prevent fluid flow in a proximal direction through the lumen (e.g., towards the bladder). Using a check valve may improve the reliability of sensed parameters and prevent air bubbles or reverse flow into the bladder.

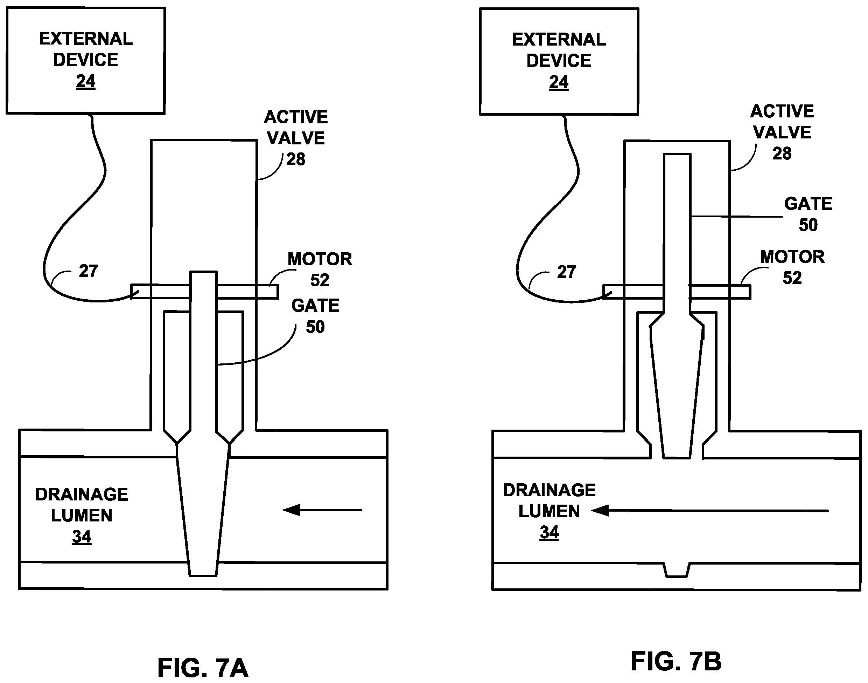

In some examples according to this disclosure, a kidney monitoring system includes a Foley catheter having two or more lumens, such as three or more lumens. In some examples in which the Foley catheter includes three or more lumens, the lumens may include a first lumen (which may be called an injection lumen or an active sampling lumen), an inflation lumen, and a drainage lumen. The inflation lumen may be configured to transport an inflation fluid from an inflation opening on a distal portion of the Foley catheter to an anchoring member of the Foley catheter. The anchoring member may be configured to hold the Foley catheter in place, such that the proximal end of the Foley catheter remains in the bladder of a patient when the anchoring member is deployed. The active sampling lumen may be fluidically coupled to a pump or similar device configured to pull a fluid from a bladder of a patient towards a sensor. In some examples, the active sampling lumen and/or the drainage lumen may also include a check valve or other valve configured to prevent or minimize the back flow (e.g., flow towards the bladder of the patient) of fluid flow and/or to prevent a vacuum from occurring in or near the bladder of the patient. In some examples, the drainage lumen and/or active sampling lumen may include a valve configured to prevent fluid flow when not sampling and facilitate fluid flow when sampling. For example, control circuitry (also referred to herein as processing circuitry, can be configured to control the valve to control whether or not the bladder is drained when not sampling fluid from the bladder using the pump. The valve can be passive or active. An example an active valve is described with reference to FIGS. 7A and 7B. Other example valves are described in U.S. Patent Application No. 16/856,937, entitled "Catheter with Valves" and filed on April 23, 2020, which is hereby incorporated by reference in its entirety.

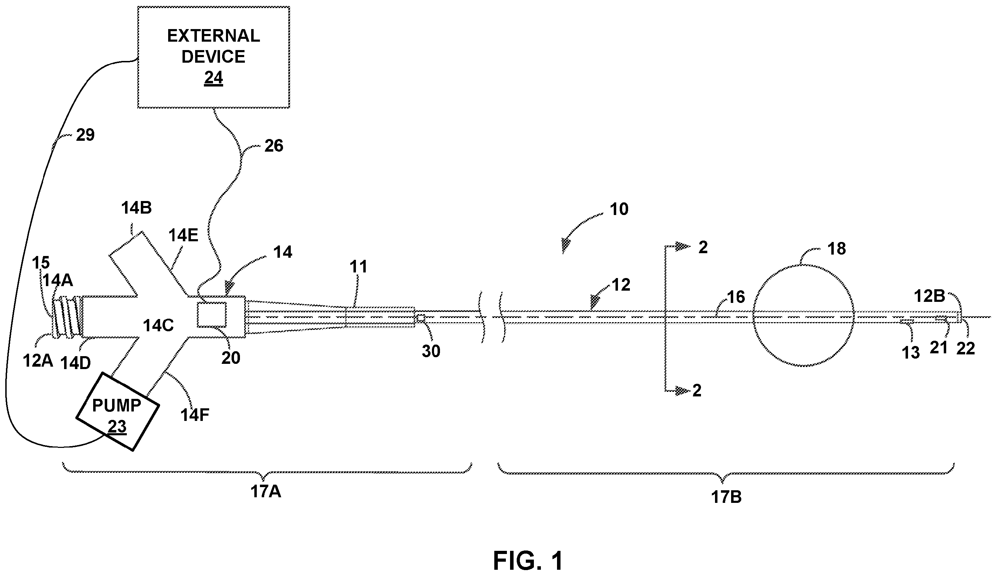

FIG. 1 is a conceptual side elevation view of an example catheter 10, which includes elongated body 12, hub 14, and anchoring member 18. Catheter 10 is a Foley catheter. While a Foley catheter and its intended use are primarily referred to herein to describe catheter 10, in other examples, catheter 10 can be used for other purposes, such as to drain wounds or for intravascular monitoring or other medical procedures.

Catheter 10 includes a distal portion 17A and a proximal portion 17B. Distal portion 17A includes a distal end 12A of elongated body 12 and is intended to be external to a body of a patient when in use, while proximal portion 17B includes a proximal end 12B of elongated body 12 and is intended to be internal to the body of the patient when in use. For example, when proximal portion 17B is positioned within a patient, e.g., such that proximal end 12B of elongated body 12 is within the bladder of the patient, distal portion 17A may remain outside of the body of the patient.

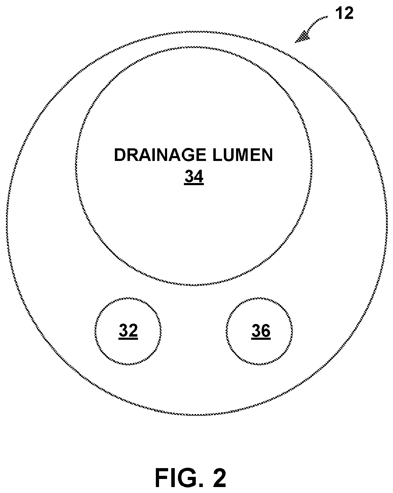

Elongated body 12 is a structure (e.g., a tubular structure) that extends from distal end 12A to proximal end 12B and defines one or more inner lumens. In the example shown in FIGS. 1-2, elongated body 12 defines lumen 32, drainage lumen 34 and inflation lumen 36 (shown in FIG. 2). In some examples, drainage lumen 34 is configured to drain a fluid from a target site, such as a bladder. In other examples drainage lumen 34 may be used for any other suitable purpose, such as to deliver a substance or another medical device to a target site within a patient. Drainage lumen 34 may extend from proximal fluid opening 13 to distal fluid opening 14A. Both fluid opening 13 and fluid opening 14A may be fluidically coupled to drainage lumen 34, such that a fluid may flow from one of fluid opening 13 or fluid opening 14A to the other of fluid opening 13 or fluid opening 14A through drainage lumen 34. Fluid opening 13 and fluid opening 14A may also be referred to as drainage openings.

In some examples, lumen 32 (shown in FIG. 2) may be an injection lumen configured to deliver a fluid to a target site, such as a bladder. In other examples, lumen 32 may be an active sampling lumen coupled to pump 23, may house sensor 20 and/or sensor 21, or may be used for any other suitable purpose, such as to deliver a medical device to a target site within a patient. Lumen 32 may extend from distal fluid opening 14C to proximal fluid opening 22. Both fluid opening 14C and fluid opening 22 may be fluidically coupled to lumen 32, such that a fluid may flow from one of fluid opening 14C or fluid opening 22 to the other of fluid opening 14C or fluid opening 22 through lumen 32. In the example where lumen 32 is an injection lumen, fluid opening 14C and fluid opening 22 may be referred to as injection openings. In the example where lumen 32 is an active sampling lumen, fluid opening 14C and fluid opening 22 may be referred to as active sampling openings. While fluid opening 22 is shown at the proximal end 12B of elongated body 12, in the examples in which fluid opening 22 exists, fluid opening 22 may be positioned elsewhere on proximal portion 17B proximal to anchoring member 18.

Pump 23 is configured to actively pull fluid from the bladder of the patient through lumen 32, e.g., directly from the bladder or from drainage lumen 34 or another lumen. In such a case, lumen 32 may direct the fluid to a dissolved oxygen sensor (e.g., an pO₂ sensor), such as sensor 20. In some examples, pump 23 may be incorporated into a sensor, such as sensor 20, or sensor 20 may be incorporated into pump 23. Pump 23 can be any suitable pump, such as, but not limited to a positive displacement pump or a centrifugal pump. Positive displacement pumps that may be used include peristaltic pumps (which may have an advantage that tubing in contact with urine may be readily disposable), gear pumps, piston pumps (which may be accompanied by a valve system, such as check valve 30, to secure one-way flow of urine out of the bladder), vane pumps, membrane pumps, plunger pumps, and the like. Positive displacement pumps may have an advantage in that they are self-priming. For example, a positive displacement pump may not depend on urine flowing due to gravity or pressure, and the flow of urine to sensor 20 may be entirely controlled by the positive displacement pump in some examples.

In some examples, Foley catheter 10 includes a mechanism to help ensure that pumping only occurs when urine is present in the bladder to minimize impacts to bladder tissue, e.g., from a pump applying a suction force (e.g., a vacuum force) to the bladder tissue. For example, sensor 21 may include a pressure sensor configured to generate a signal indicative of an amount of suction force applied to the bladder, and control circuitry of external device 24 (or another device) may be configured to control operation of pump 23 based on the signal from the pressure sensor. As an example, the control circuitry can be configured to shut off pump 23 in response to determining, based on the signal from the pressure sensor, that the suction force is greater than or equal to a threshold value, which can indicate the bladder is empty or nearly empty.

In another example, sensor 21 may include a flow sensor configured to generate a signal indicative of an amount of flow of a fluid (e.g., a fluid flow rate) in drainage lumen 34, and control circuitry of external device 24 (or another device may be configured to control operation of pump 23 based on the signal from the flow sensor. As an example, the control circuitry can be configured to turn on pump 23 in response to determining, based on the signal from the flow sensor, that the flow of the fluid in the drainage lumen 34 is greater than or equal to a predetermined threshold value and to shut off pump 23 in response to determining, based on the signal from the flow sensor, that the flow of the fluid in drainage lumen 34 is less than a predetermined threshold value. This may, for example, enable pump 23 to pump fluid through lumen 32 (or another lumen) when the urine output of the patient is greater than or equal to a threshold rate and to not pump when urine output of the patient is relatively low. The control circuitry can, for example, be connected to pump 23 via connection 29, which may be a wired or wireless connection.

In addition to or instead of the pressure and/or the fluid flow sensor, in some examples, the system can include an ultrasound sensor configured to generate a signal that changes as a function of a volume of urine in the bladder, and the control circuitry of device 24 can be configured to control pump 23 based on the signal from the ultrasound sensor. Inflation lumen 36 (shown in FIG. 2) is configured to transport a fluid, such as sterile water or saline, or a gas, such as air, from inflation opening 14B to anchoring member 18. For example, an inflation device (not shown) may pump fluid or gas into inflation lumen 36 through inflation opening 14B into anchoring member 18 such that anchoring member 18 is inflated to a size suitable to anchor catheter 10 within the patient's bladder. In examples in which anchoring member 18 does not include an expandable balloon, rather than defining inflation lumen 36, elongated body 12 may define an inner lumen configured to receive a deployment mechanism (e.g., a pull wire or a push wire) for deploying an expandable structure anchoring member 18 and hub 14 may comprise fluid opening 14A, fluid opening 14C and an opening 14B via which a clinician may access the deployment mechanism. Alternatively, in some examples in which anchoring member 18 does not include an expandable balloon, catheter 10 may be a two lumen catheter, rather than a three lumen catheter.

In some examples, elongated body 12 has a suitable length for accessing the bladder of a patient through the urethra. The length may be measured along central longitudinal axis 16 of elongated body 12. In some examples, elongated body 12 may have an outer diameter of about 12 French to about 14 French, but other dimensions may be used in other examples. Distal portion 17A and proximal portion 17B of elongated body 12 may each have any suitable length.

In the example shown in FIG. 1, distal end 12A of elongated body 12 is received within hub 14 and is mechanically connected to hub 14 via an adhesive, welding, or another suitable technique or combination of techniques. Hub 14 is positioned at a distal end of elongated body 12 and defines an opening through which the one or more inner lumens (e.g., lumen 32, drainage lumen 34 and inflation lumen 36, shown in FIG. 2) of elongated body 12 may be accessed and, in some examples, closed. While hub 14 is shown in FIG. 1 as having three arms, 14D, 14E and 14F, hub 14 may have any suitable number of arms, which may depend on the number of inner lumens defined by elongated body 12. For example, each arm may be fluidically coupled to a respective inner lumen of elongated body 12. In the example of FIG. 1, hub 14 comprises a fluid opening 14A, which is fluidically coupled to drainage lumen 34, an inflation opening 14B, which is fluidically coupled to inflation lumen 36, and a fluid opening 14C which is fluidically coupled to lumen 32 (shown in FIG. 2) of elongated body 12.

A fluid collection container (e.g., a urine bag or more rigid container) may be attached to fluid opening 14A for collecting urine draining from the patient's bladder. Inflation opening 14B may be operable to connect to an inflation device to inflate anchoring member 18 positioned on proximal portion 17B of catheter 10. Anchoring member 18 may be uninflated or undeployed when not in use. Hub 14 may include connectors, such as connector 15, for connecting to other devices, such as the fluid collection container and the inflation source. Fluid opening 14C may be configured to connect to an injection device or pull device, such as pump 23, for injecting fluid into the patient's bladder or for pulling fluid out of patient's bladder, respectively. In some examples, catheter 10 includes strain relief member 11, which may be a part of hub 14 or may be separate from hub 14.

Sensor 20 may be positioned on distal portion 17A, such as on hub 14. In some examples, sensor 20 is alternatively positioned distal to distal end 12A, such as on additional tubing or another structure connected to hub 14. In some examples, sensor 20 may be positioned within lumen 32 or outside of lumen 32 but in fluid communication with lumen 32. Sensor 20 is configured to sense dissolved oxygen, in a fluid, such as urine. The fluid can be, for example, fluid in drainage lumen 34 and/or lumen 32 or fluid received from drainage lumen 34 and/or lumen 32.

Proximal portion 17B of catheter 10 comprises anchoring member 18, fluid opening 13, fluid opening 22 and, in some examples, sensor 21. In some examples, sensor 21 is contained within drainage lumen 34 or lumen 32. Anchoring member 18 may include any suitable structure configured to expand from a relatively low profile state to an expanded state in which anchoring member 18 may engage with tissue of a patient (e.g., inside a bladder) to help secure and prevent movement of proximal portion 17B out of the body of the patient. For example, anchoring member 18 can include an anchor balloon or other expandable structure. When inflated or deployed, anchoring member 18 may function to anchor catheter 10 to the patient, for example, within the patient's bladder. In this manner, the portion of catheter 10 on the proximal side of anchoring member 18 may not slip out of the patient's bladder. Fluid opening 13 may be positioned on the surface of elongated body 12 between anchoring member 18 and the proximal end 12B (as shown) or may be positioned at the proximal end 12B. Fluid opening 22 may be positioned at the proximal end 12B (as shown) of elongated body 12 or may be positioned on the surface of elongated body between anchoring member 18 and the proximal end 12B.

In the example of FIG. 1, distal portion 17A of catheter 10 includes check valve 30 and sensor 20. Check valve 30 may be positioned in or about drainage lumen 34 or in or about lumen 32 (both shown in FIG. 2). Check valve 30 is configured to minimize the back flow (e.g., flow towards the bladder of the patient) of fluid flow through the lumen 32 and/or 34 in a direction away from sensor 20. Check valve 30 may, therefore, help fluid flow past sensor 20 to enable sensor 20 to sense the dissolved oxygen content (or other parameter of interest) of the fluid. As used herein, "passing", "pass", or "past", is intended to include the instance where the fluid flows proximate sensor 20 to enable sensor 20 to generate a signal indicative of the parameter of interest of the fluid, and is not intended to be interpreted as the fluid bypassing the sensor.

Check valve 30 may have any suitable configuration. For example, check valve 30 can include a duckbill valve, a swing valve, a ball valve, a diaphragm valve, an umbrella valve, or any other type of check valve. While in the example of FIG. 1, check valve 30 is shown as positioned on distal portion 17A of elongated body 12, check valve 30 may be positioned anywhere on elongated body 12, such as on distal portion 17A or proximal portion 17B.

Sensor 20 is configured to sense dissolved oxygen, in a fluid, such as urine. Sensor 20 may be positioned on hub 14, as shown, in lumen 32 or in drainage lumen 34, elsewhere on distal portion 17A of the body of catheter 10, or may be positioned distal to distal end 12A, e.g., on tubing connected to a fluid collection container (e.g., a urine bag) or the like. In some cases, sensor 20 may be one or more sensors that are relatively larger, require relatively more electrical, optoelectrical, or optical connections, than sensors that could be located on the proximal portion 17B. While sensor 20 is primarily discussed herein as sensing dissolved oxygen (e.g., oxygen tension or uPO₂) and/or fluid output, in some examples, sensor 20 may additionally include sensor(s) configured to sense one or more of flow rate, temperature, pressure, fluid concentration, amount of dissolved carbon dioxide in the fluid, turbidity, fluid pH, fluid color, fluid creatinine, and/or motion.

In some examples, sensor 20 is mechanically connected to elongated body 12 or another part of catheter 10 using any suitable technique, such as, but not limited to, an adhesive, welding, by being embedded in elongated body 12, via a crimping band or another suitable attachment mechanism or combination of attachment mechanisms. As discussed above, in some examples, sensor 20 is not mechanically connected to elongated body 12 or catheter 10, but is instead mechanically connected to a structure that is distal to a distal end of catheter 10, such as to tubing that extends between hub 14 and a fluid collection container.

Sensor 20 may be configured to communicate sensor data to an external device 24. External device 24 may be a computing device, such as a workstation, a desktop computer, a laptop computer, a smart phone, a tablet, a server or any other type of computing device that is configured to receive, process and/or display sensor data. Sensor 20 may communicate sensor data to the external device via a connection 26. Connection 26 may be an electrical, optical, wireless or other connection.

Although only sensor 20 and sensor 21 are shown in FIG. 1, in other examples, catheter 10 can include any suitable number of sensors on proximal portion 17B and any suitable number of sensors on distal portion 17A, where the sensors on proximal portion 17B sense the same or different parameters and the sensors on distal portion 17A sense the same or different parameters. In addition, some or all of the sensors on proximal portion 17B can sense the same or different parameters as the sensors on distal portion 17A. For example, in the case where sensors on the distal portion may be temperature dependent, it may be desirable to sense temperature both on the proximal portion 17B and the distal portion 17A.

Elongated body 12 may be structurally configured to be relatively flexible, pushable, and relatively kink- and buckle- resistant, so that it may resist buckling when a pushing force is applied to a relatively distal portion of elongated body 12 to advance the elongated body 12 proximally through the urethra and into the bladder. Kinking and/or buckling of elongated body 12 may hinder a clinician's efforts to push the elongated body proximally.

In some examples, at least a portion of an outer surface of elongated body 12 includes one or more coatings, such as an anti-microbial coating, and/or a lubricating coating. The lubricating coating may be configured to reduce static friction and/ kinetic friction between elongated body 12 and tissue of the patient as elongated body 12 is advanced through the urethra.

In some examples, at least a portion of an inner surface of one or more lumens, such as lumen 32 or drainage lumen 34, or an outer surface of elongated body 12, may be coated with a coating that is impermeable or relatively impermeable (as compared with the material(s) of elongated body 12) to a substance of interest, such as oxygen. Such a coating may include polyvinyl chloride, polyvinylidene chloride, ethylene vinyl alcohol, metalization of the surface, or the like. In some examples, the coating may be similar to or the same as the coating(s) described in U.S. Patent Application Publication No. 2021/0186428, entitled "Catheter Including a Plurality of Sensors," filed on April 21, 2020, and claiming the benefit of U.S. Provisional Patent Application 62/952,776, filed on December 23, 2019, both of which are hereby incorporated by reference in their entirety.

FIG. 2 is a conceptual diagram illustrating an example cross-section of elongated body 12 of catheter 10, where the cross-section is taken along line 2-2 in FIG. 1 in a direction orthogonal to central longitudinal axis 16. FIG. 2 depicts a cross section of elongated body 12, which defines lumen 32, drainage lumen 34, and inflation lumen 36. While lumen 32, drainage lumen 34, and inflation lumen 36 are shown as circular in cross-section, they may have any suitable cross-sectional shape in other examples, such as oval, rectangular, or the like. As noted above, in some examples, lumen 32 may be referred to as an injection lumen or an active sampling lumen.

Elongated body 12 can define any suitable number of lumens. For example, although one inflation lumen 36 is shown in FIG. 2, in other examples, elongated body 12 can define a plurality of inflation lumens 36, e.g., that are distributed around lumen 32 or drainage lumen 34. As another example, anchoring member 18 may be an expandable structure that is not an inflatable balloon. In such examples, inflation lumen 36 may be replaced by a deployment mechanism which may permit a clinician to expand the expandable structure. For example, inflation lumen 36 may be replaced by a mechanical device that may be pushed and pulled separately from the catheter 10 by a clinician to expand or retract the expandable structure. As another example of a different lumen configuration, in some examples, elongated body 12 may not include lumen 32 and can have only drainage lumen 34 and inflation lumen 36.

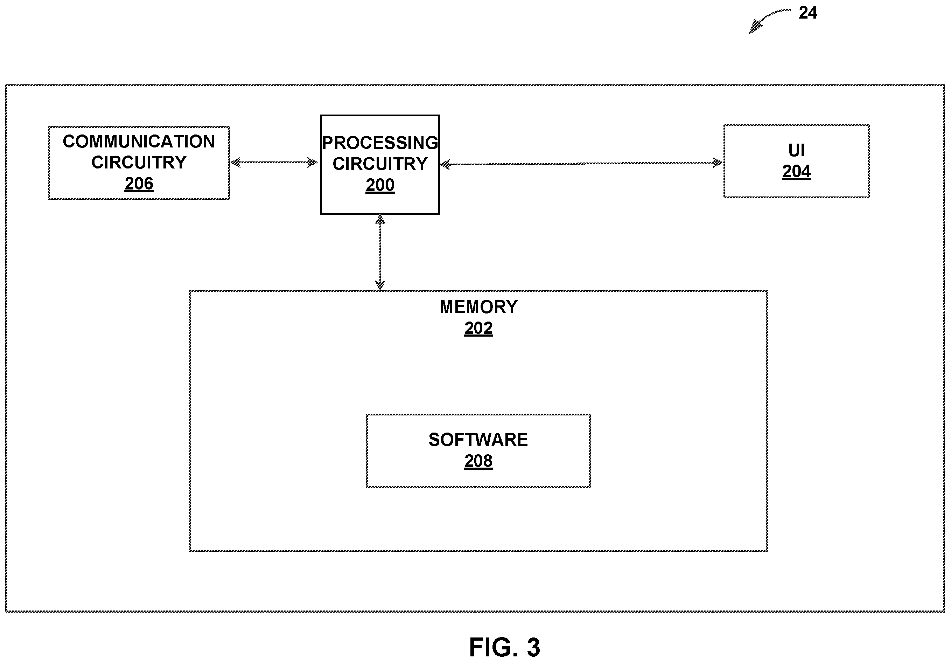

FIG. 3 is a functional block diagram illustrating an example of an external device 24 configured to communicate with sensor 20, receive information (e.g., via an electrical or optical signal) from sensor 20, and/or generate an output based on a dissolved oxygen content sensed by sensor 20 and indicated by the information received from sensor 20. In some examples, external device 24 also is configured to communicate with or receive information from sensor 21. In the example of FIG. 3, external device 24 includes processing circuitry 200, memory 202, user interface (UI) 204, and communication circuitry 206. In some examples, processing circuitry 200 may also be referred to as and include control circuitry. External device 24 may be a dedicated hardware device with dedicated software for the reading sensor data. Alternatively, external device 24 may be an off-the-shelf computing device, e.g., a desktop computer, a laptop computer, a tablet, or a smartphone running a mobile application that enables external device 24 to read sensor data from sensor 20.

In some examples, a user of external device 24 may be a clinician or a patient. In some examples, a user uses external device 24 to monitor a patient's kidney function. For example, sensor 20 (FIG. 1) may output a signal indicative of a dissolved oxygen content in a fluid (e.g., urine) via connection 26 (FIG. 1) to external device 24. Processing circuitry 200 may determine the dissolved oxygen content based on the signal and may generate an output based on the determined dissolved oxygen content, which is presented to a user via UI 204, which may display the measure of dissolved oxygen content. The output can be, for example, a quantitative value or qualitative indication of the dissolved oxygen content, a graphical, textual, or other indication of a kidney function of the patient, an indication of a likelihood of AKI, or any combination thereof.

In some examples, the user may interact with external device 24 via UI 204, which may include a display to present a graphical user interface to the user and/or sound generating circuitry configured to generate audio output, and a keypad or another mechanism (such as a touch sensitive screen) configured to receive input from the user. External device 24 may communicate with sensor 20, sensor 21, or pump 23 (FIG. 1) using wired, wireless or optical methods through communication circuitry 206. For example, processing circuitry 200 of external device 24 may process sensor data from sensor 20 or sensor 21. In some examples, processing circuitry 200 is configured to control pump 23 to turn off pump 23 when the bladder of the patient is relatively empty and/or when the bladder is outputting urine at a rate lower than a predetermined threshold, and turn on pump 23 when the bladder of the patient is relatively full and/or when the bladder is outputting urine at a rate greater than or equal to the predetermined threshold. In this manner, processing circuitry 210 may help protect bladder tissue of the patient from the suction force generated by pump 23, while reducing the transit time through catheter 10 of the fluid when the bladder of the patient is relatively full, thereby reducing any oxygen permeation that may occur during transit of the fluid through catheter 10.

Processing circuitry 200, as well as other processing and control circuitry described herein, may include any combination of integrated circuitry, discrete logic circuity, analog circuitry, such as one or more microprocessors, digital signal processors (DSPs), application specific integrated circuits (ASICs), or field-programmable gate arrays (FPGAs). In some examples, processing circuitry 200 includes multiple components, such as any combination of one or more microprocessors, one or more DSPs, one or more ASICs, or one or more FPGAs, as well as other discrete or integrated logic circuitry, and/or analog circuitry.

Memory 202 may store program instructions, such as software 208, which may include one or more program modules, which are executable by processing circuitry 200. When executed by processing circuitry 200, such program instructions may cause processing circuitry 200 and external device 24 to provide the functionality ascribed to them herein. The program instructions may be embodied in software and/or firmware. Memory 202 may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media.

This disclosure describes techniques and devices configured to aid in the monitoring of the one or both kidneys of a patient. In some examples, processing circuitry 200 of external device 24 monitors the amount of oxygen dissolved in the urine uPO₂ in the bladder as it has been shown that this measurement reflects the oxygenation of the kidneys. To do this the urine output (rate of urine production) and the amount of oxygen dissolved in the urine may be measured. Example techniques of this disclosure utilize a Foley catheter 10 with one or more sensors configured to generate outputs indicative of these parameters. In some examples, the sensors are part of the Foley catheter 10. In other examples, the sensors are not part of the Foley catheter 10.

To sense the uPO₂ at distal end 12A or distal portion 17A of Foley catheter 10 (away from the patient), care should be taken to minimize or even prevent oxygen from diffusing from the external environment into the urine in drainage lumen 34, which may confound the sensed signal or diffusing out of the urine into the external environment. Urine monitoring systems described herein can be configured to utilize active sampling methods to pull the urine from the bladder such that uPO₂ can be sensed without the confounding effects of oxygen diffusion through the Foley catheter that may occur at the relatively low flow rates that can be observed for urine output for catheterized patients.

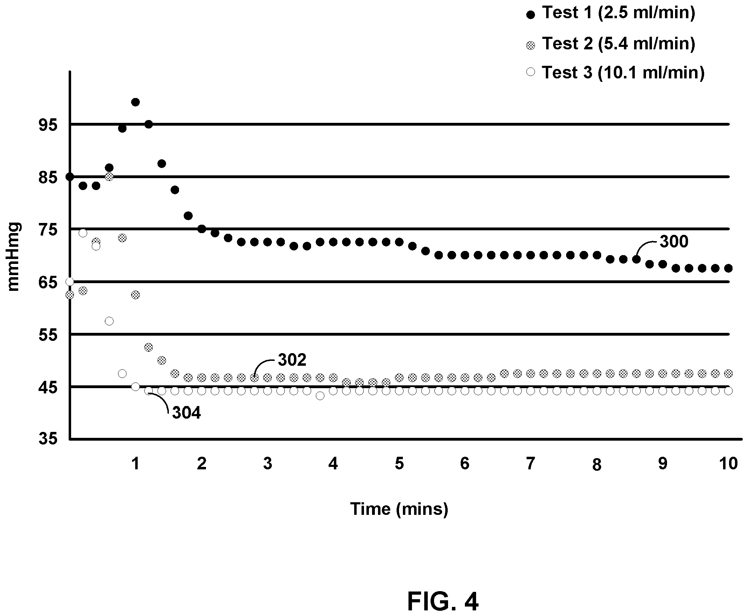

FIG. 4 is a graph illustrating pO₂ measurements of water that was initially set to ~43 mmHg and allowed to flow through a silicone Foley catheter at different flow rates. As shown in FIG. 4, the O₂ pick up from water with an initial pO₂ of ~43 mmHg flowing through a silicone Foley Catheter at a flow rate of 2.5 milliliters/minute (ml/min) (Test 1 whose measurements shown as black filled circles 300) measured a pO₂ uptake of 26.3 mmHg. The O₂ pick up from water with an initial pO₂ of ~43 mmHg flowing through a silicone Foley Catheter at a flow rate of 5.4 ml/min (Test 2 whose measurements shown as grey filled circles 302) measured a pO₂ uptake of 6.4 mmHg. The O₂ pick up from water with an initial pO₂ of ~43 mmHg flowing through a silicone Foley Catheter at a flow rate of 10.1 ml/min (Test 3 whose measurements shown as white filled circles line 304) measured a pO₂ pick up of 3 mmHg. Some urine flow rates range from 0-5 ml/min for catheterized patients. Hence, the pO₂ pick up could be significant for silicone catheters, particularly at relatively low flow rates. Therefore, in some cases, a relatively high flow rate (e.g., greater than or equal to a flow rate in a range of 5 ml/min to 10 ml/min), or a maximized flow rate, may be desirable to minimize diffusion of oxygen into urine flowing through one or more lumens of a Foley catheter.

Patients can be catheterized during and after major surgery using an indwelling urinary (Foley) catheter (e.g., catheter 10) inserted into the bladder via the urethra. In some examples, oxygen may be measured at the distal end of the inserted urinary catheter using an oxygen sensor (e.g., sensor 20) inserted in the flow stream between the catheter and the urine collecting container. As mentioned above, commercially available Foley catheters are oxygen permeable in varying degree depending on the catheter design, including at least materials and dimensions. This results in diffusion of oxygen between the urine in the catheter and the ambient air as well as the urethra over the catheter wall. Furthermore, the catheter wall constitutes an oxygen buffer which takes up/releases oxygen from/to the urine. These mechanisms can result in an alteration of the oxygen partial pressure (pO₂) from the true value in the bladder over the length of the catheter to the sample point at the distal end of the catheter where the pO₂ is measured. The degree of equilibration between the oxygen in the urine and the oxygen surrounding the catheter can be affected by one or more of: 1) the catheter material; 2) the outer diameter and inner diameter(s) of the catheter, the inner diameters referring to, for example, the size of the lumens; 3) the wall thickness of the catheter; 4) the length of the catheter; 5) the portion of the catheter situated in the urethra and in the ambient air, respectively; 6) the flow speed of the urine - a relatively high flow speed results in a short transit time and thus, lower equilibration with the exterior oxygen concentration; 7) the change in flow speed; 8) the change in the oxygen partial pressure in the urine; or 9) temperature.

In general, silicone Foley catheters have a relatively high oxygen permeability resulting in a relatively high degree of oxygen equilibration with the oxygen at the outer wall of the catheter. Latex Foley catheters have a lower, but still potentially significant, oxygen equilibration with the oxygen at the outer wall of the catheter. Latex also has the disadvantage that some patients are sensitive to Latex, and as a result, many hospitals may not allow medical devices that include latex. PVC Foley catheters have the lowest oxygen permeability, but have the draw back that they are typically stiffer than the silicone and latex catheters, which may result in a lower degree of patient comfort and convenience.

Due to the relatively high oxygen permeability of Foley catheters, it may be desirable for measurement of urine oxygenation to be performed as close to the kidneys as possible to obtain the best signal, e.g., more accurate reading indicative of the oxygenation status of the kidneys. However, it may not be easy to place a sensor through the ureter, so an alternative location to use is in the bladder. The measurements may also be taken outside of the body, but the urine transit through a Foley catheter has the possibility of changing the measurement. For example, silicone is a common material used in Foley catheters. Silicone has a relatively high permeability to oxygen. In some cases, the urine oxygenation is in the range of 10 to 50 mmHg, which is substantially lower than the atmospheric level of about 159 mmHg at sea level, creating a gradient that may drive atmospheric oxygen into the urine in the lumens of the Foley catheter. Therefore, it may be desirable to use active sampling of urine to increase the flow rate of the urine passing a sensor, such as a dissolved oxygen sensor.

A urine monitoring system described herein is configured to apply active sampling of a fluid of interest (e.g., urine) with the use of a pump, such as pump 23, or other device, to actively pull the fluid through a collection device or through a lumen of catheter 10. For example, a pump can be used with a triple lumen Foley catheter 10 to actively pull the urine from the bladder through a lumen 32 and/or drainage lumen 34 to sensor 20 (which is a pO₂ sensor) at a flow rate of greater or equal to 10 ml/min such that the urine has a relatively short transit time through lumen 32 or drainage lumen 34 (compared to the transit time that would be observed without the pump and with the fluid flowing via gravity) to minimize oxygen diffusion through walls of catheter 10 into the urine to mitigate against artificially inflating the uPO₂.

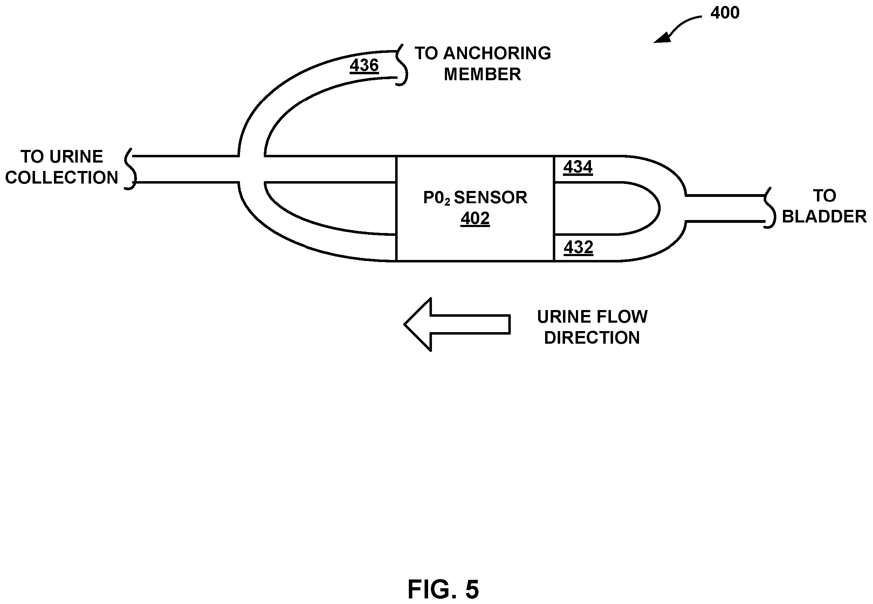

FIG. 5 is a conceptual diagram of an example urine monitoring system including a pO₂ sensor 402 coupled to a Foley catheter 400. The example of FIG. 5 depicts a portion of Foley catheter 400 which may be an example of Foley catheter 10 of FIG. 1. Sensor 402 can be an example of sensor 20 of FIG. 1. While generally described as a triple lumen Foley catheter, in some examples, the Foley catheter of FIG. 5 may define more than three lumens. Foley catheter 400 defines active sampling lumen 432, which may be an example of lumen 32 of FIG. 2, and drainage lumen 434, which may be an example of drainage lumen 432 of FIG. 2. Foley catheter 400 may also define inflation lumen 436, which may be an example of inflation lumen 36 of FIG. 2.

As shown in FIG. 5, in some examples, both drainage lumen 434 and active sampling lumen 432 may distally connect to a sensor (pO₂ sensor 402, which may also be referred to as a dissolved oxygen sensor), though in other examples, only active sampling lumen 432 may connect to pO₂ sensor 402. While not shown in FIG. 5, a pump, such as pump 23 is coupled to Foley catheter 400 distal to pO₂ sensor 402 or may be integrated with pO₂ sensor 402 and be configured so as to direct fluid to pO₂ sensor 402 at a higher flow rate than otherwise may be, for example at a flow rate greater than or equal to 10ml/min. For example, rather than relying on only gravity or other natural (passive) forces to direct urine from a bladder to sensor 402 positioned outside of the bladder, pump 23 is configured to actively cause the fluid to flow at a higher flow rate to sensor 402 in a direction away from the bladder. That is, pump 23 pumps fluid through lumen 432 and/or lumen 434 to sensor 402. In this way, pump 23 actively draws fluid through lumen 432 and/or lumen 434 to enable sensor 402 to actively sense the parameter of interest in the fluid.

With the aid of the motive forces from pump 23, fluid, such as urine, may flow from a position proximal to pO₂ sensor 402 from the bladder of the patient to a position distal to pO₂ sensor 402. In this way, pump 23 facilitates the active sensing of the dissolved oxygen content of the fluid by pO₂ sensor 402. The fluid may flow into urine collection tubing or directly into a collection container distal to the distal end (not shown) of Foley catheter 400.

In some examples, pO₂ sensor 402 may only sense the dissolved oxygen content of the urine that has been sampled at a faster flow rate (e.g., greater than or equal to 10 ml/min). While not shown in FIG. 5, in some examples, pO₂ sensor 402 may only sense the dissolved oxygen content of the urine that is separated from drainage lumen 434. In other words, in such examples, pO₂ sensor 402 may only sense urine from or in active sampling lumen 432. In such examples, drainage lumen 434 may be fluidically isolated from pO₂ sensor 402.

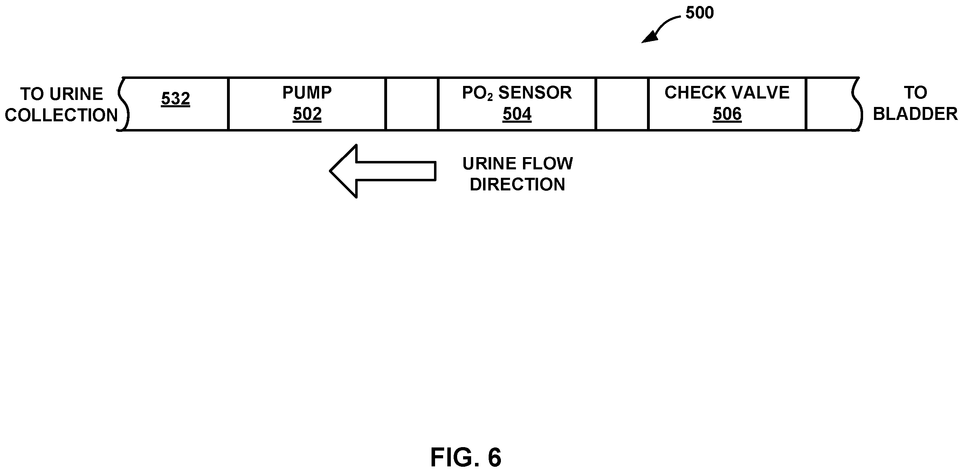

FIG. 6 is a conceptual diagram of another example urine monitoring system including an example pO₂ sensor within a lumen of a Foley catheter. The example of FIG. 6 shows Foley catheter 500, which defines an active sampling lumen 532. Foley catheter 500 may be an example of Foley catheter 10 of FIG. 1 or Foley catheter 400 of FIG. 5. Active sampling lumen 532 may be an example of lumen 32 of FIG. 2 or active sampling lumen 432 of FIG. 5. pO₂ sensor 504 is a dissolved oxygen sensor.

In the example of FIG. 6, check valve 506 is located in or about active sampling lumen 432. Check valve 506 is configured to prevent the back flow of urine towards the bladder, and to facilitate only the actively sampled urine being sensed or measured by pO₂ sensor 504. For example, check valve 506 can be configured to open to enable fluid flow towards sensor 504 only in response to a flow rate through lumen 532 greater than or equal to a threshold rate. The threshold flow rate may be selected to be greater than an expected passive flow of fluid out of the bladder through lumen 532 in the absence of suction forces from pump 502. Check valve 506 may, therefore, provide a passive mechanism to only enable to fluid to flow to sensor 504 at a flow rate greater than or equal to a desired "active sampling" flow rate. Check valve 506 is configured to close in response to the fluid flow slowing to below the threshold flow rate.

In some examples, check valve 506 is also configured to open in response to a pressure greater than or equal to a pressure threshold being applied to a proximal side of check valve 506, which may result when bladder is relatively full. This may help reduce patient discomfort from the relatively full bladder, even in the absence of active pumping of urine through lumen 532 by pump 502.

In some examples, pO₂ sensor 504 may be contained within a relatively small container located within active sampling lumen 532, for example, to keep a location of pO₂ sensor 504 relatively stable and from entering pump 502.

Pump 502 may be an example of pump 23 of FIG. 1. Pump 502 may be located in or about active sampling lumen 532. Pump 502 may be located distal to pO₂ sensor 504 and check valve 506, as shown. In this manner, pump 502 may pull fluid, such as urine, through check valve 506 and direct the fluid to pO₂ sensor 504, to direct the fluid to pO₂ sensor 504 such that pO₂ sensor 504 may sense the dissolved oxygen content of the fluid. Pump 502 may be configured to pull fluid at a rate that exceeds 10mL/minute, thereby reducing any confounding effect oxygen diffusing from the environment into the urine or from the urine into the environment may have on the transiting fluid.

In some examples, pO₂ sensor 504 may sample a relatively small amount of fluid, for example between 50 microliters (µl) and 5 ml. For example, if Foley catheter 500 is around 30 centimeters (cm) long and active sampling lumen 532 (or alternatively a drainage lumen if pO₂ sensor 504 is located in a drainage lumen) is 0.1 cm in diameter then pO₂ sensor 504 may sense approximately 0.236 cm³ (π*0.05²*30) or approximately 0.236 ml of fluid. In another example, if Foley catheter 500 is around 18 cm long and active sampling lumen 532 (or alternatively a drainage lumen if pO₂ sensor 504 is located in a drainage lumen) is 0.5 cm in diameter then pO₂ sensor 504 may sense approximately 3.5 cm³ (π*0.25²*18) or approximately 3.5 ml of fluid.

In the example of FIG. 6, Foley catheter 500 may define a distal end at or near the urine collection location and a proximal end configured to be positioned closer to a bladder of a patient than the distal end. Pump 502 is located closer to the distal end than pO₂ sensor 504, as shown.

FIGS. 7A and 7B are conceptual diagrams of an example catheter including an active valve located in or about a drainage lumen of the catheter. While described as being located in or about drainage lumen 34 (FIG. 1), in some examples, active valve 28 may alternatively, or additionally, be located in or about lumen 32 (FIG. 1). In the example of FIG. 7A, active valve 28 is a gate valve having a motor 52 and gate 50. While active valve is a gate valve in this example, active valve may be a gate valve, a globe valve, a plug valve, a ball valve, a butterfly valve, a diaphragm valve, a pinch valve, a needle valve or any type of valve that may enable the flow of a fluid through drainage lumen 34 when open and prevent the flow of the fluid through drainage lumen 34 when closed. In FIG. 7A, active valve 28 is closed, preventing the flow of a fluid, such as urine, through drainage lumen 34. In this example, gate 50 is positioned in drainage lumen 34 blocking the flow of the fluid through drainage lumen 34. When the catheter shown in FIGS. 7A and 7B is in the patient and positioned to drain fluid from a bladder of a patient, and when active valve is closed, urine is held in the bladder and is not flowing through drainage lumen 34, e.g., to a fluid collection container.

External device 24 is configured to control the opening and closing of active valve 28 to control when fluid is drained from the bladder of a patient. For example, processing circuitry 200 of device 24 can be configured to open valve 28 periodically and after a sufficient level of urine flow (e.g., greater than or equal to a threshold flow rate) is detected in drainage lumen 32, e.g., using a flow sensor.

In the example shown in FIGS. 7A and 7B, external device 24 is connected to motor 52 of active valve 28. In response to determining that a first condition occurred (e.g., pump 23 being activated, a clinician input to external device 24, or a signal from a pressure sensor, such as sensor 21), processing circuitry 200 of external device 24 may control active valve 28 to open. For example, processing circuitry 200 may transmit a first signal over connection 27 to motor 52 of active valve 28 to open gate 50. Motor 52 may then open (e.g., raise) gate 50 in response to receiving the first signal. Connection 27 can include a wired connection, an optical connection or a wireless connection.

In the example of FIG. 7B, active valve 28 is in the open position, enabling the flow of fluid, such as urine, through drainage lumen 34 and, for example, out of a bladder of a patient to a collection container or the like when elongated body 12 is positioned such that proximal end 12B is positioned in the bladder. In this example, gate 50 is open (e.g., raised) and is not substantially blocking fluid flow through drainage lumen 34, thereby enabling the flow of the fluid through drainage lumen 34. Such an implementation may prevent or lessen the chance that a vacuum builds up in the bladder of the patient while pump 23 is operating.

In response to determining that a second condition has occurred (e.g., pump 23 being deactivated, a clinician input to external device 24, or a signal from a pressure sensor, such as sensor 21), processing circuitry 200 may control valve 28 to close. For example, processing circuitry 200 may transmit a second signal over connection 27 to motor 52 to close gate 50. Motor 52 may then close gate 50 in response to receiving the second signal bringing the state of active valve 28 back to the example of FIG. 7A. In the closed position, gate 50 blocks more fluid flow through drainage lumen 34 more than when gate 50 is in the open position. That is, more fluid is permitted to flow through lumen 34 when gate 50 is in the open position than when gate 50 is in the closed position. In some examples, gate 50 blocks substantially all fluid flow through drainage lumen 34 (e.g., all fluid flow or a majority of fluid flow to the extent permitted by manufacturing tolerances) when gate 50 is in the closed position.

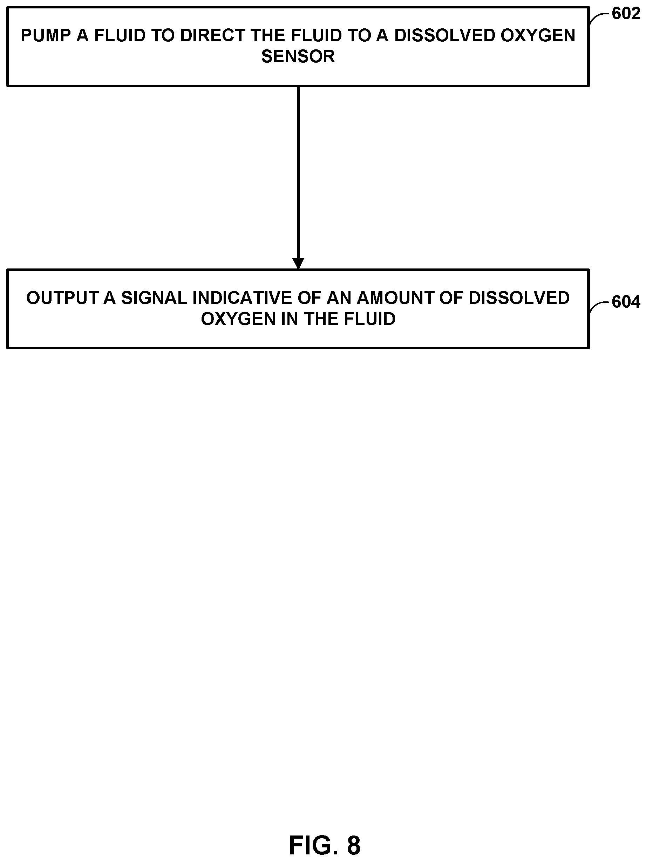

FIG. 8 is a flow diagram illustrating an example active sampling technique. While the active sampling technique of FIG. 8 is described with respect to Foley catheter 500 of FIG. 6, this technique may be performed by other devices or systems. In the technique shown in FIG. 8, pump 502 pumps a fluid to direct the fluid to dissolved oxygen sensor 504 (602). For example, pump 502 may pump urine to pull the urine past dissolved oxygen sensor 504 enabling dissolved oxygen sensor 504 to sense the dissolved oxygen content in the fluid. In some examples, pump 502 is configured to pump the fluid at a rate greater than or equal to 10 milliliters per minute (10ml/min). This may reduce that transit time of the fluid when compared to the fluid naturally draining, for example being pulled by gravity or otherwise being naturally expelled by the bladder of the patient.

Dissolved oxygen sensor 504 actively samples the fluid. For example, dissolved oxygen sensor 504 may sense a parameter of the fluid being pumped, such as an amount of dissolved oxygen. Dissolved oxygen sensor 504 may output a signal indicative of an amount of dissolved oxygen in the fluid (604). For example, dissolved oxygen sensor 504 may output the signal via connection 26 (FIG. 1) to processing circuitry 200 of external device 24 (FIGS. 1 and 3).

In some examples, dissolved oxygen sensor 504 is located in a lumen defined by a Foley catheter. For example, dissolved oxygen sensor 504 may be located in active sampling lumen 532 of Foley catheter 500. In some examples, dissolved oxygen sensor 402 (FIG. 5) is configured to generate the signal indicative of the amount of dissolved oxygen in the fluid from a plurality of lumens (e.g., active sampling lumen 432 and drainage lumen 434) defined by Foley catheter 400.

In some examples, pump 502 is located further from the source of the fluid than the dissolved oxygen sensor 504. In some examples, the fluid includes urine from a bladder of a patient. In some examples, control circuitry (such as processing circuitry 200 of FIG. 4) receives a signal from a pressure sensor (e.g., sensor 21) indicative of pressure in the bladder, and controls pump 502 to stop pumping fluid from the bladder based on the signal from the pressure sensor. In some examples, check valve 506 prevents back flow of the fluid, such as urine back toward the bladder. In some examples, the fluid is urine. In some examples, pump 502 and dissolved oxygen sensor 504 are coupled to a triple lumen Foley catheter.

By employing a pump to pump a fluid and actively sampling the pumped fluid, the techniques of this disclosure may reduce a transit time of the fluid through one or more catheter lumens. Reducing the transit time of the fluid through one or more catheter lumens may improve the accuracy of sensed parameters of the fluid as there is less time for the parameters to be confounded due to the parameters diffusing from the environment into the urine or from the urine into the environment.

Any of the techniques or examples described herein may be used alone or in combination with one or more other techniques or examples.

The techniques described in this disclosure, including those attributed to sensor 20, sensor 21, processing circuitry 200, communication circuitry 206, UI 204 or various other components, may be implemented, at least in part, in hardware, software, firmware or any combination thereof. For example, various aspects of the techniques may be implemented within one or more processors, including one or more microprocessors, DSPs, ASICs, FPGAs, or any other equivalent integrated or discrete logic circuitry. The term "processor" or "processing circuitry" may generally refer to any of the foregoing logic circuitry, alone or in combination with other logic circuitry, or any other equivalent circuitry.

Such hardware, software, firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

When implemented in software, the functionality ascribed to the systems, devices and techniques described in this disclosure may be embodied as instructions on a computer-readable medium such as RAM, ROM, NVRAM, EEPROM, FLASH memory, magnetic data storage media, optical data storage media, or the like. The instructions may be executed to support one or more aspects of the functionality described in this disclosure.

Various examples have been described. These and other examples are within the scope of the following claims.

## Claims

1. A system comprising:
a Foley catheter (10, 400, 500);
a dissolved oxygen sensor (20, 402, 504) located in a lumen (34, 432, 434, 532) defined by the Foley catheter (10, 400, 500); and
a pump (23, 502) configured to pump urine from a bladder of a patient to direct the urine to the dissolved oxygen sensor (20, 402, 504), the dissolved oxygen sensor (20, 402, 504) configured to output a signal indicative of an amount of dissolved oxygen in the urine,
wherein the Foley catheter (10, 400, 500) defines a distal end and a proximal end, the proximal end configured to be positioned closer to a bladder of a patient than the distal end is to the bladder of the patient, the pump (23, 502) located closer to the distal end than the dissolved oxygen sensor (20, 402, 504) is to the distal end.

2. The system of claim 1, wherein the signal is indicative of the amount of dissolved oxygen in the urine from a plurality of lumens (432, 424) defined by the Foley catheter (400).

3. The system of claim 1, wherein the Foley catheter is a triple lumen Foley catheter, wherein the pump (23) and the dissolved oxygen sensor (20) are coupled to the triple lumen Foley catheter.

4. The system of claim 1, further comprising:
a valve configured to move between a closed state in which, when a proximal end of the Foley catheter is positioned in a bladder of a patient, the valve is configured to retain fluid in the bladder, and an open state in which the valve enables drainage of fluid from the bladder via the lumen.

5. The system of any of claims 1-4, wherein the pump (23) is configured to pump the fluid at a rate greater than or equal to 10 milliliters per minute.

6. The system of any of claims 1-5, further comprising:
a pressure sensor (21) configured to generate a signal indicative of pressure in a bladder of a patient or a signal from a flow sensor indicative of flow of the fluid; and
control circuitry configured to control the pump (23) to stop pumping fluid from the bladder based on the signal from the pressure sensor (21) or the signal from the flow sensor.

7. The system of any of claims 1-6, further comprising a check valve configured to prevent back flow of the fluid in a direction away from the dissolved oxygen sensor (20).

## Patentansprüche

1. System, umfassend:
einen Foley-Katheter (10, 400, 500);
einen Sensor (20, 402, 504) für gelösten Sauerstoff, der sich in einem Lumen (34, 432, 434, 532) befindet, das durch den Foley-Katheter (10, 400, 500) definiert ist; und
eine Pumpe (23, 502), die konfiguriert ist, um Urin aus einer Blase eines Patienten zu pumpen, um den Urin zu dem Sensor für gelösten Sauerstoff (20, 402, 504) zu leiten, wobei der Sensor für gelösten Sauerstoff (20, 402, 504) konfiguriert ist, um ein Signal auszugeben, das eine Menge an gelöstem Sauerstoff in dem Urin angibt,
wobei der Foley-Katheter (10, 400, 500) ein distales Ende und ein proximales Ende definiert, wobei das proximale Ende konfiguriert ist, um näher an einer Blase eines Patienten positioniert zu sein als das distale Ende an der Blase des Patienten ist, wobei sich die Pumpe (23, 502) näher an dem distalen Ende befindet als der Sensor (20, 402, 504) für gelösten Sauerstoff an dem distalen Ende ist.

2. System nach Anspruch 1, wobei das Signal aus einer Vielzahl von Lumen (432, 424), die durch den Foley-Katheter (400) definiert sind, die Menge an gelöstem Sauerstoff in dem Urin angibt.

3. System nach Anspruch 1, wobei der Foley-Katheter ein dreilumiger Foley-Katheter ist, wobei die Pumpe (23) und der Sensor (20) für gelösten Sauerstoff mit dem dreilumigen Foley-Katheter gekoppelt sind.

4. System nach Anspruch 1, ferner umfassend:
ein Ventil, das konfiguriert ist, um sich zwischen einem geschlossenen Zustand, in dem das Ventil, wenn ein proximales Ende des Foley-Katheters in einer Blase eines Patienten positioniert ist, konfiguriert ist, um Flüssigkeit in der Blase zurückzuhalten, und einem offenen Zustand zu bewegen, in dem das Ventil ein Abfließen von Flüssigkeit aus der Blase über das Lumen ermöglicht.

5. System nach einem der Ansprüche 1 bis 4, wobei die Pumpe (23) konfiguriert ist, um die Flüssigkeit mit einer Rate von größer als oder gleich 10 Millilitern pro Minute zu pumpen.

6. System nach einem der Ansprüche 1 bis 5, ferner umfassend:
einen Drucksensor (21), der konfiguriert ist, um ein Signal, das den Druck in einer Blase eines Patienten angibt, oder ein Signal von einem Durchflusssensor, das den Durchfluss der Flüssigkeit angibt, zu erzeugen; und
eine Steuerschaltung, die konfiguriert ist, um die Pumpe (23) zu steuern, um das Pumpen von Flüssigkeit aus der Blase basierend auf dem Signal von dem Drucksensor (21) oder dem Signal von dem Durchflusssensor zu stoppen.

7. System nach einem der Ansprüche 1 bis 6, ferner umfassend ein Rückschlagventil, das konfiguriert ist, um einen Rückfluss der Flüssigkeit in eine Richtung weg von dem Sensor (20) für gelösten Sauerstoff zu verhindern.

## Revendications

1. Système comprenant :
une sonde de Foley (10, 400, 500) ;
un capteur d'oxygène dissous (20, 402, 504) situé dans une lumière (34, 432, 434, 532) définie par la sonde de Foley (10, 400, 500) ; et
une pompe (23, 502) conçue pour pomper l'urine d'une vessie d'un patient afin de diriger l'urine vers le capteur d'oxygène dissous (20, 402, 504), le capteur d'oxygène dissous (20, 402, 504) étant configuré pour émettre un signal indiquant une quantité d'oxygène dissous dans l'urine,
dans lequel la sonde de Foley (10, 400, 500) définit une extrémité distale et une extrémité proximale, l'extrémité proximale étant conçue pour être positionnée plus près d'une vessie d'un patient que l'extrémité distale ne l'est de la vessie du patient, la pompe (23, 502) étant située plus près de l'extrémité distale que le capteur d'oxygène dissous (20, 402, 504) ne l'est de l'extrémité distale.

2. Système selon la revendication 1, dans lequel le signal indique la quantité d'oxygène dissous dans l'urine à partir d'une pluralité de lumières (432, 424) définies par la sonde de Foley (400).

3. Système selon la revendication 1, dans lequel la sonde de Foley est une sonde de Foley à triple lumière, dans lequel la pompe (23) et le capteur d'oxygène dissous (20) sont accouplés à la sonde de Foley à triple lumière.

4. Système selon la revendication 1, comprenant en outre :
une valve conçue pour se déplacer entre un état fermé dans lequel, lorsqu'une extrémité proximale de la sonde de Foley est positionnée dans une vessie d'un patient, la valve est conçue pour retenir le fluide dans la vessie, et un état ouvert dans lequel la valve permet le drainage du fluide de la vessie par le biais de la lumière.

5. Système selon l'une quelconque des revendications 1-4, dans lequel la pompe (23) est conçue pour pomper le fluide à un taux supérieur ou égal à 10 millilitres par minute.

6. Système selon l'une quelconque des revendications 1-5, comprenant en outre :
un capteur de pression (21) configuré pour générer un signal indiquant la pression dans une vessie d'un patient ou un signal d'un capteur de débit indiquant le débit du fluide ; et
un circuit de commande configuré pour commander la pompe (23) afin d'arrêter le pompage du fluide de la vessie en fonction du signal du capteur de pression (21) ou du signal du capteur de débit.

7. Système selon l'une quelconque des revendications 1-6 comprenant en outre un clapet anti-retour conçu pour empêcher le reflux du fluide dans une direction opposée au capteur d'oxygène dissous (20).
